# EUROPEAN PATENT SPECIFICATION

(11) **EP 3 074 070 B1**
(45) Date of publication and mention of the grant of the patent: **17.07.2019**
(21) Application number: 14803233.7
(22) Date of filing: 25.11.2014
(51) Int. Cl.: A61M 5/32

(54) **INJECTOR DEVICES**
INJEKTOREN
INJECTEURS

(30) Priority: 26.11.2013 GB 201320837
(43) Date of publication of application: 05.10.2016
(73) Proprietor: Owen Mumford Limited, Oxfordshire OX20 1TU (GB)
(72) Inventor: FINCHAM, Sam Kevin, Woodstock Oxfordshire OX20 1TU (GB); VARDÉ, Andrew John, Woodstock Oxfordshire OX20 1TU (GB); SMITH, Clive Kenneth, Woodstock Oxfordshire OX20 1TU (GB)
(74) Representative: White, Duncan Rohan
(86) International application number: PCT/GB2014/053488
(87) International publication number: WO 2015/079219

(56) References cited:
- EP-A1- 1 743 668
- WO-A1-2011/068542
- WO-A1-2012/049147
- WO-A2-2009/107945
- US-A1- 2008 108 951
- US-B1- 7 001 364

## Description

### FIELD OF THE INVENTION

The invention relates to substance delivery devices and, in particular, caps for such devices, including injection devices such as auto-injectors and pen injectors.

### BACKGROUND OF THE INVENTION

Automatic injection devices are routinely used in the medical field to deliver a measured dose of medicine to a user. Due to their user friendly design, they can be safely used by patients for self-administration, although in some circumstances they may be used by trained personnel. They may be designed to be carried by the user for use at any time, in which case they should be as small and inconspicuous as possible to improve user compliance. Automatic injection devices for the self-administration of parenteral drugs include single dose and multi dose reusable and disposable auto-injectors and pen injectors (e.g. insulin pens), which are suitable for a wide range of primary containers, including pre-filled glass and plastic syringes and pre-filled cartridges.

A typical automatic injection device comprises several parts which may include; a syringe containing medicine, a needle fixed to the end of the syringe, a firing mechanism including a spring (or possibly other drive means such as an electric motor or gas drive means), and a trigger. The spring may be preloaded, or may be set using a dose setting mechanism such as a dial. The firing mechanism is activated by the trigger and forces the medicine through the needle and into the user. A mechanical lock may be provided to prevent the trigger from being accidentally pressed. This could be, for example, simply a catch that must be moved out of the way in order to access the trigger. Alternatively the device may have no internal drive means but may be operated by pushing, for example, a knob at one end of the device.

There are many different types of auto-injector, for example, disposable injectors, which are discarded when a cartridge containing the medicament is exhausted and reusable injectors, which can be reset when a cartridge containing the medicament is exhausted. In each case the injectors may be repeatable pre-set dose injectors that permit the initial setting of a required dose and can thereafter be used for the setting of only that dose, or they may be variable dose injectors that allow the user to adjust the dosage for each injection.

EP-A-1954337 discloses, a pen-type injector having a user operable dose setting mechanism. The injector comprises a casing and a cap, which snap fit together. A dose knob is disposed at one end of the casing. A trigger is also disposed on an outer surface of the casing. A cartridge housing is secured to the end of the casing opposite to the dose knob. The cartridge housing is arranged to receive a disposable medicine filled cartridge. Such a cartridge has a rubber bung sealing one end of the cartridge, with the other end being arranged to receive a disposable needle. The cartridge is typically multi-use, that is to say each cartridge contains multiple doses of medicine. A user attaches a new needle to the cartridge for each injection, and disposes of the cartridge or the whole device after all of the doses have been used.

As previously noted, the needle should be replaced before each new injection procedure, for example by discarding the needle safely directly after an injection has been performed. With a pen and cap combination where the cap cannot accommodate the needle then it will be necessary to remove the needle before the cap can be assembled onto the pen body.

However there may be circumstances where the user wishes to temporarily retain a used needle on the device, for instance when a suitable container is not immediately available for the safe disposal of the needle. WO2009107945 discloses a prior art injector in which the length of the cap can be altered via sleeves.

If the cap is sized to accommodate a needle then the overall length of the pen-injector is longer and the device may be cumbersome for the user. If the cap is sized so as not to accommodate a needle then there is no possibility for the user to retain a needle on the device, whether before or after use, for whatever reason.

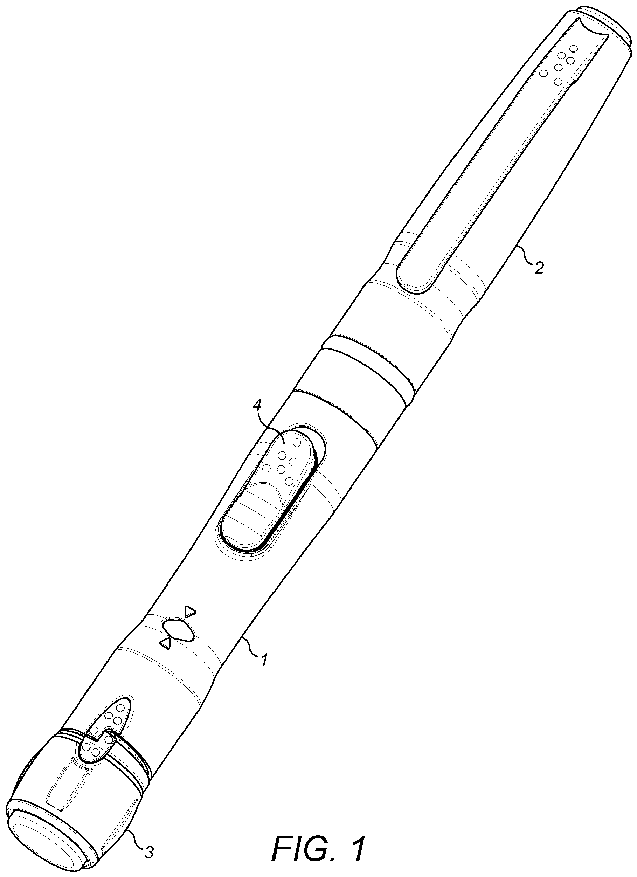

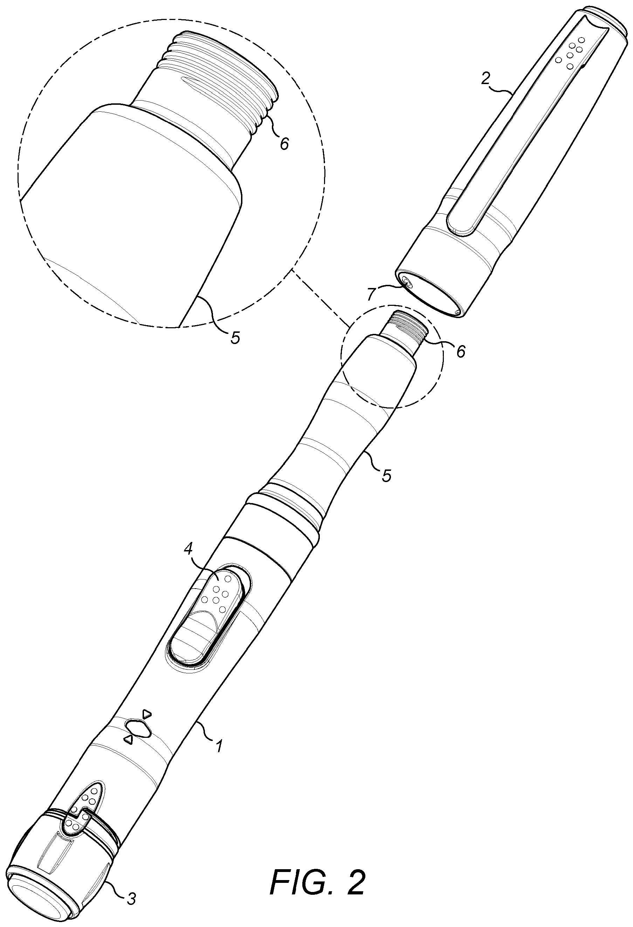

Figures 1 and 2 show a device very similar to those described in EP-A-1954337. Figures 1 and 2 show an auto-injector pen comprising a casing 1 and a cap 2, which snap fit together. A comparison between figure 1 and 2 indicates how the cap 2 sits on the housing 1. A dose knob 3 is disposed at one end of the casing 1. A trigger 4 is also disposed on an outer surface of the casing 1. A cartridge housing 5 is secured to the end of the casing opposite to the dose knob 3. The cartridge housing 5 is arranged to receive a disposable medicine filled cartridge as for the device described above in connection with EP-A-1954337.

As can be seen from figure 2 the cap is a snap fit onto the cartridge housing 5. Protrusions 7 on the inner wall of the cap 2 co-operate with ridges or grooves 8 on the external wall of the housing 5.

At the end 6 of the cartridge housing 5 distal from the dose knob 3 is indicated a screw thread for receiving a needle assembly (not shown and referred to as a needle hereinafter).

EP-A-1743668 discloses a device having a protection portion attached to a flexible joint portion which is connected to a base. The base is joined to the retainer of an injection needle.

US-A-2008108951 discloses a storage system for a needleless pen delivery device syringe and a hypodermic needle adapted for attachment to and use with the pen delivery device syringe. A tubular sleeve has a first hollow chamber for receiving therein the needleless pen delivery device syringe, and a hollow cap has a second hollow chamber for receiving therein the hypodermic needle. The cap is adapted to affix to the sleeve to form a continuous tubular housing, and the sleeve is adapted to receive the pen delivery device syringe only when the needle is not attached to the syringe

US-B-7001364 discloses a device for shielding a needle to prevent accidental needle sticks. The device contains two shields that surround the needle, one of which attaches to the needle hub, and the second which extends beyond the tip of the needle. The second shield slides into the first shield thereby exposing the needle during injection. The device also contains a spring that holds the second shield in the extended position except when compressed, and a locking mechanism that secures the second shield automatically after injection in the extended position except when unlocked by the user prior to injection. The spring ensures that the second shield remains in the extended position when mechanism is unlocked, shielding the needle, except when the spring is compressed by pressure against the injection site, and when the needle is withdrawn from the injection site, the second shield is pushed back and automatically locked into place for disposal of the needle.

### SUMMARY

The invention provides a re-usable, multi-dose injection device according to claim 1.

The two caps may use the same mechanical means to attach to the needle receiving end of the device.

The two caps may use different mechanical means to attach to the needle receiving end of the device.

At least one cap may use a snap fit to attach to the needle receiving end of the device.

At least one cap may use a co-operating screw thread arrangement to attach to the needle receiving end of the device.

### BRIEF DESCRIPTION OF DRAWINGS

Figure 1 is a perspective view of a prior art pen injector.
Figure 2 is an exploded view and enlarged view of the pen injector of figure 1a.
Figure 3 is a perspective view of an injector device according to the invention;
Figure 4 shows two side views of a cap for an injector device in two different configurations;
Figure 5a shows two side views of a cap for an injector device in two different configurations;
Figure 5b is a cross-section of the cap of figure 5a;
Figure 6a shows two side views of a cap for an injector device in two different configurations;
Figure 6b is a cross-section of the cap of figure 6a;
Figure 7a shows two cross-sectional views of a cap for an injector device in two different configurations;
Figure 7b is enlarged cross-sectional view of a portion of the cap of figure 7a with a helical spring;
Figure 7c is a detailed side view of a component of the cap of figure 7a;
Figure 8a shows two side views of a cap for an injector device in two different configurations on a housing of an injector device;
Figure 8b is a cross section of one of the configurations shown in figure 8a;
Figures 9a, 9b,and 9c are side views of a cap and injector device housing according to an aspect of the invention.

### DETAILED DESCRIPTION

The present invention, as defined in the independent claim, is suitable for use with any re-usable injector, such as a multi-dose device that is suitable for receiving a medication cartridge, and for receiving a needle. The trigger and dose knob of figures 1 and 2 are both optional features. In figures 1 and 2 the injector is shown provided with a single cap that is able to accommodate the needle. A specific example of a needle would be one complying with ISO 11608-2:2012, which specifies requirements and test methods for single-use, double-ended, sterile needles for needle-based injection systems (NISs) that fulfill the specifications of ISO 11608-1.

According to the invention the injector is supplied with two caps one sized to accommodate the needle and one that is shorter and which may not be able to accommodate the needle. It would be necessary to remove the needle, which should be disposed of after use in any case, before replacing the longer cap with the shorter cap. The two caps may use the same attachment mechanism. In the embodiment the caps both snap fit onto the housing. In an alternative embodiment the two caps are affixed to the housing using different means. For example the longer cap might snap fit onto the housing and the shorter cap might be applied to a screw thread that is also used for affixing the needle.

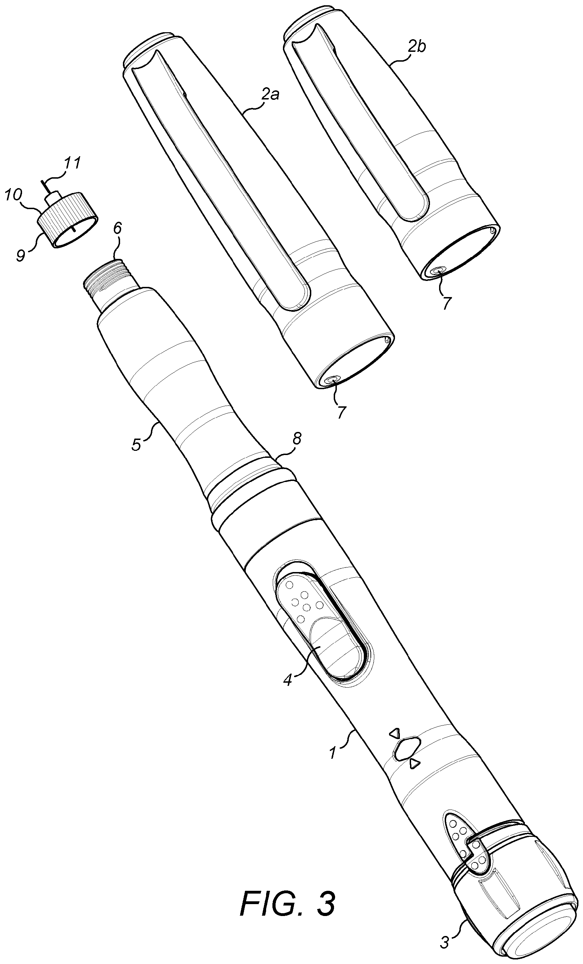

An embodiment is shown in figure 3 of an auto-injector pen supplied with 2 caps: a longer cap 2a able to accommodate a needle 9 and a short cap 2b that is, for example, 21mm shorter than the longer cap 2a. The short cap 2b can be used when a user chooses not to leave needles attached to the housing 5. When the user has used the injector device the needle 9 can be unscrewed from the housing 5. Otherwise the device is substantially the same as the device shown in figures 2a and 2b.

The two caps 2a and 2b are each snap fits onto the cartridge housing 5. Protrusions 7 on the inner wall of each cap 2a, 2b co-operate with ridges or grooves 8 on the external wall of the housing 5.

Whilst in embodiments of the invention a cap may be described or shown as a 'snap-fit', this is not intended to be limiting. In each case the cap could be remove-ably attached to the body by any convenient means including a screw connection, bayonet type coupling, or other suitable means known to those skilled in the art.

In the case where the shorter cap uses the same fixing mechanism as the needle; the larger cap could be designed to accommodate the shorter cap so that when not in use both caps could be retained on the injector housing.

At least one of the caps may be removably attached to either end of the injector so that at least one cap can be conveniently retained on the injector when not in use to cover the needle end of the injector.

In another aspect, not according to the invention a single cap may be extendible in order to accommodate a needle if it is not desired to remove a needle for any reason. This allows the convenience of being able to choose a short cap length when it is not necessary to accommodate a needle within the cap and a long cap length when it is desired to accommodate a needle. This solution has the further advantage of a single cap preventing the possibility of mislaying a cap that is not in use.

There are several mechanical solutions for providing an extensible cap in accordance with the invention. The cap may have two portions connected by a screw thread, unwinding the screw thread connection would serve to increase the cap length. Alternatively a simple sliding engagement between two or more portions of the cap would serve to increase the cap length. In each case a suitable mechanical mechanism could be provided for fixing temporarily the two cap portions in the extended position and the retracted position respectively.

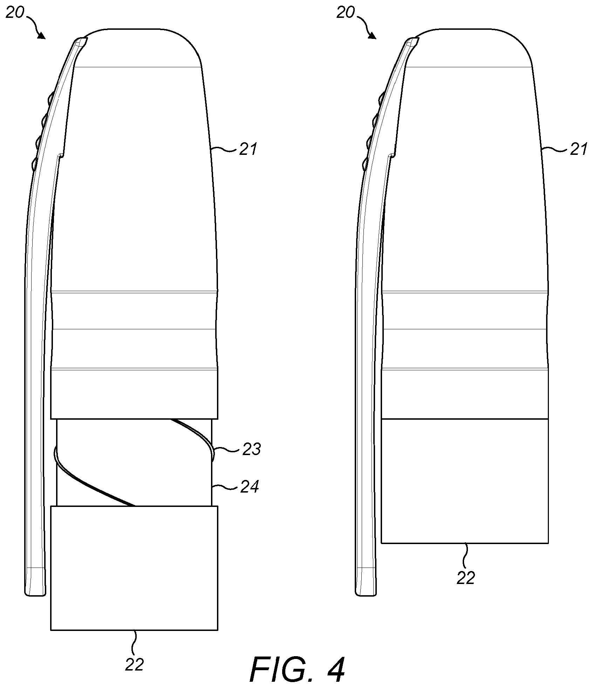

A two part extensible cap is shown in figure 4. In figure 4 the same cap is shown in extended and retracted configurations. The cap 20 includes two portions, upper portion 21 and lower portion 22 connected by a screw thread 23 on a connecting portion 24 of the upper portion 21. The screw thread 23 may be a shallow screw thread as shown co-operating with corresponding grooves (not shown) on the internal surface of lower portion 22. Of course the connecting portion 24 could be on the lower portion 22. Furthermore, the location of the screw thread 23 and groove could also be reversed. The lower portion 22 may carry the required snap fit connection means or other means for connecting the cap to the housing of an injector device.

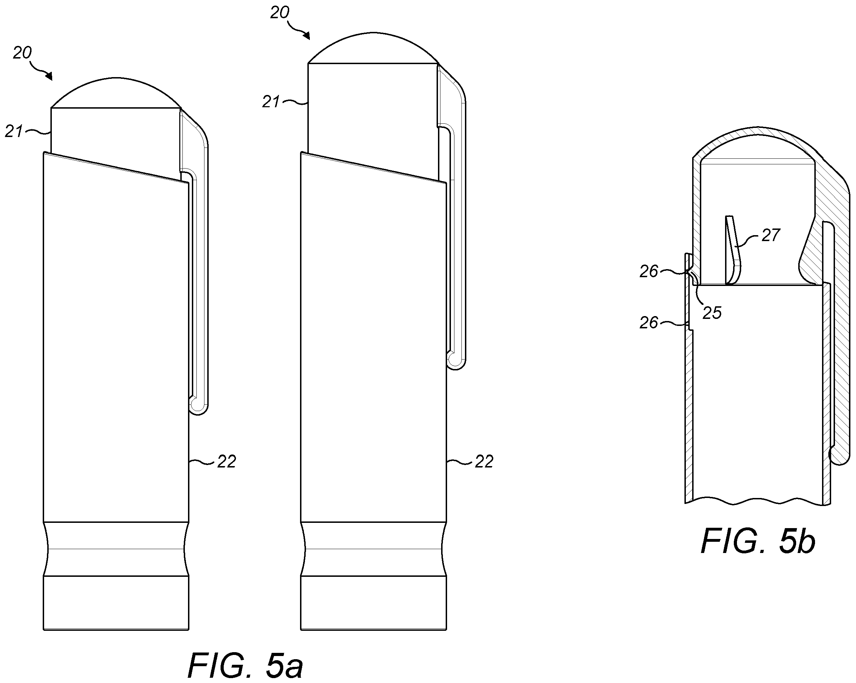

In figure 5a, a cap 20 is shown in two configurations: retracted and extended. Instead of a screw connection, the two portions 21, and 22 of the cap are slidably engaged. The two portions 21 and 22, can be moved manually from the extended position to the retracted position if no needle is present on the injector device. If a needle is present on the device the two portions 21 and 22 slide relative to each other to extend the cap 20 to accommodate the needle.

As shown in figure 5b, cooperating features on surfaces of the portions 21 and 22 latch the two portions in the retracted and extended positions. In this case there are protrusions 25 on the external surface of the upper portion 21 and corresponding recesses 26 on the internal surface of the lower portion 22. In a variation the recesses 26 are on the upper portion 21 and the protrusions 25 are on the lower portion 22. Furthermore, the upper portion 21 is shown as slidably received within the lower portion 22, whereas the upper portion 21 could receive the lower portion 22 with corresponding changes to the latching mechanism, if present.

Figure 5b also shows internal ribs 27 on an internal surface of the upper portion 21 which serve to locate on a housing of a needle if present; when the cap 20 in its retracted state is placed on an injector housing, on which a needle is present, the ribs 27 will locate on an uppermost needle housing surface 10 (see figure 3). Further pressure applied to the cap 20 will force the two portions 21 and 22 to slide relative to each other overcoming the latching force provided by cooperating features 25 and 26. The two portions 21, 22 will slide until the portions are latched in the extended position.

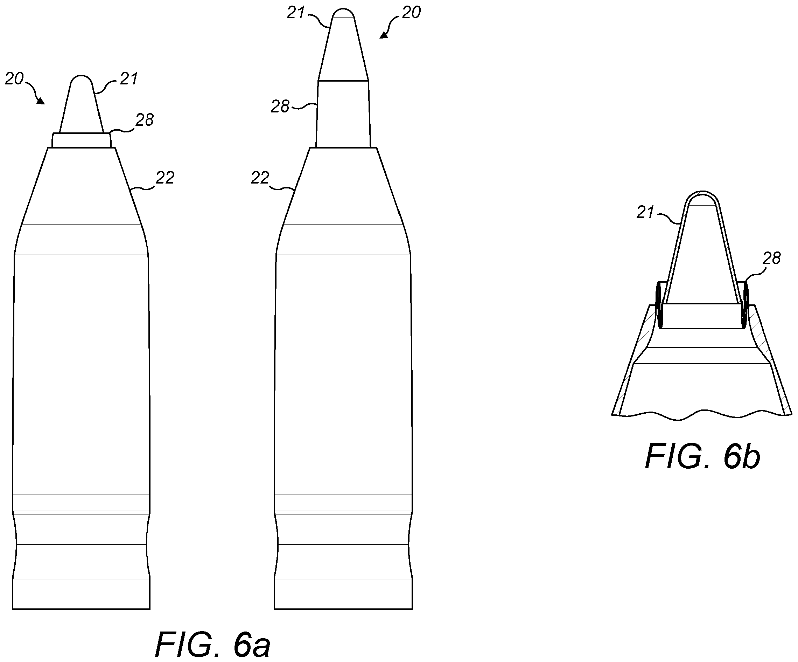

In figure 6a a cap 20 is shown in two configurations retracted and extended. An upper or tip portion 21 of the cap 20 is connected to the lower portion 22 by a flexible section 28. In the retracted position the flexible section is folded in on itself as shown in figure 6b. Thus the rigid tip 21 which may be made of plastic material can be extended by unfolding the flexible section 28 if a needle is present on the injector device when the cap 20 is placed on the device. The flexible section 28 may be made of an elastomeric material such as a silicone elastomer.

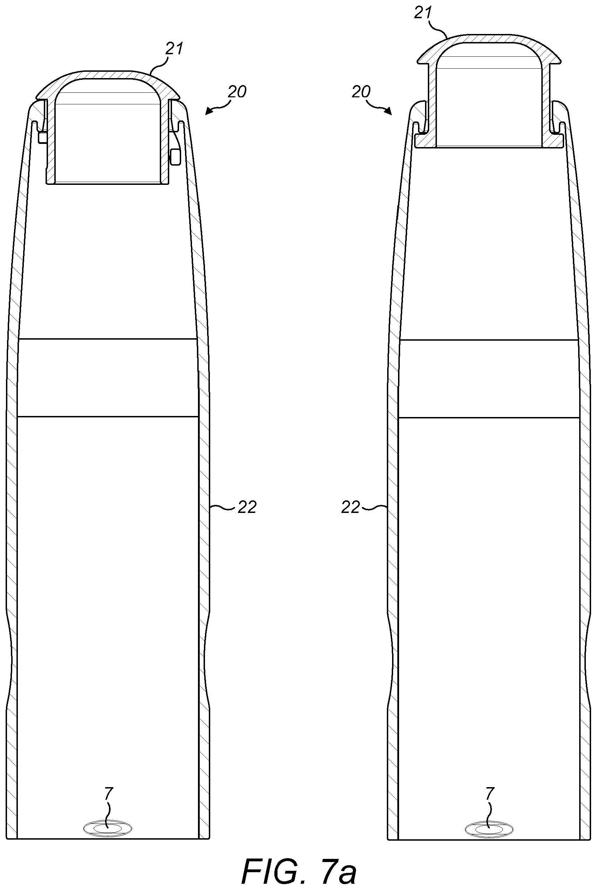

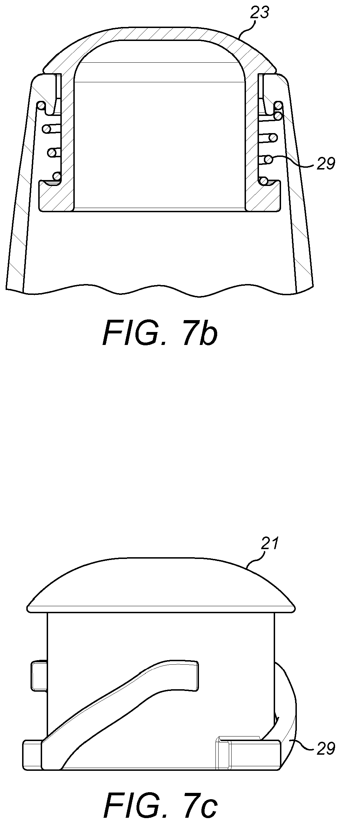

In figure 7a a cap 20 is shown in two configurations retracted and extended. In this case the upper or tip portion 21 of the cap 20 is a sprung cap portion 21. The sprung cap portion can be extended when fitting the cap to an injector device with a needle present. When no needle is present on the injector device a spring 29 biases the cap portion 21 towards the retracted position. The spring 29 may be a metal or plastic spring. The spring 29 and cap portion 21 may be formed as a single part either from a single material or as a composite moulding. Figure 7b shows a separate spring 29 in more detail, whereas figure 7c shows a single component including the cap portion 21 and the spring portion 29.

Similar to the device of Figure 6, the lower surface of the cap portion 21 may engage with the upper surface 10 of a needle 9 (see figure 3) to cause extension of the cap portion 21 so as to prevent damage to a cannula 11 when replacing the cap 20.

Whilst the embodiment of figure 7 includes a spring biasing the two cap portions 21 and 22 in a retracted position the two portions 21, 22 could be biased in an extended position. The cap 20 could be manually manipulated to provide a shorter cap configuration. Latching features could be provided to retain the cap 20 in the shorter configuration against the bias of the spring. The cap 20 could be manually manipulated to provide the cap 20 in the extended configuration against the force of the latching mechanism and features may be provided to ensure that the cap 20 is extended when the cap 20 is placed on an injector device with a needle present on the cartridge housing 5.

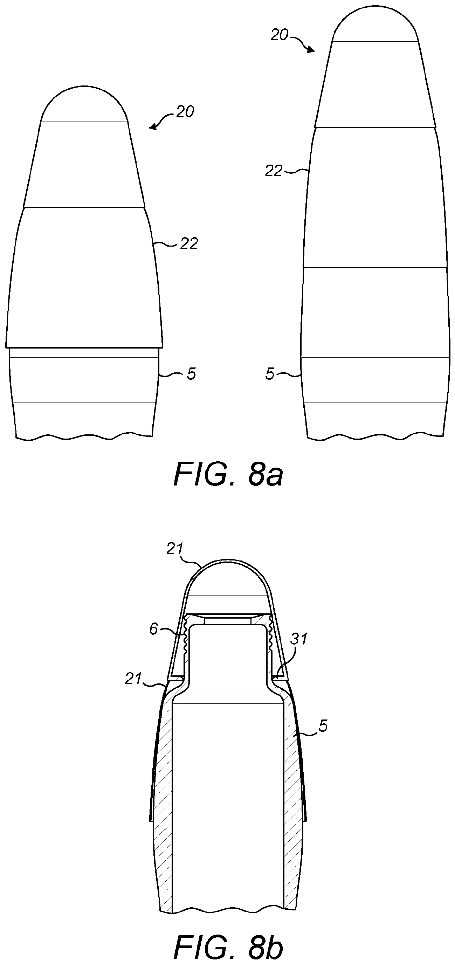

In figure 8a a cap 20 is shown having a cap portion 21 and a flexible skirt portion 22. The skirt portion 22 of the cap 20 is configured to stretch over the barrel of housing 5 of the injector device and the cap is held in place by the flexible skirt portion 22. The cap portion 21 is a rigid portion that protects the needle. The cap portion 21 can be held in a lower position if no needle is present on the injector device. If a needle is present on the device the cap portion is held in a raised position as shown in the right hand side of figure 8a.

As shown in figure 8b, when no needle is present on the housing 5, a lip 31 of the cap portion 21 engages with a surface of the housing 5 when the cap 20 is in a lowered position. When a needle connected to the end 6 of the cartridge housing 5 is present on the injector device the lip 31 engages with a surface of the needle to prevent lowering of the cap 20 which is held in a raised position protecting the cannula 11 (figure 3).

In an alternative not shown, the cap could have upper and lower portions connected by a flexible portion similar to that shown in figure 8, where the lower portion would carry means for fixing to the injector housing such as protrusions or other features co-operating with features on the housing.

In another aspect not according to the invention a single cap is provided which can be fitted in two positions on the housing of the injector pen.

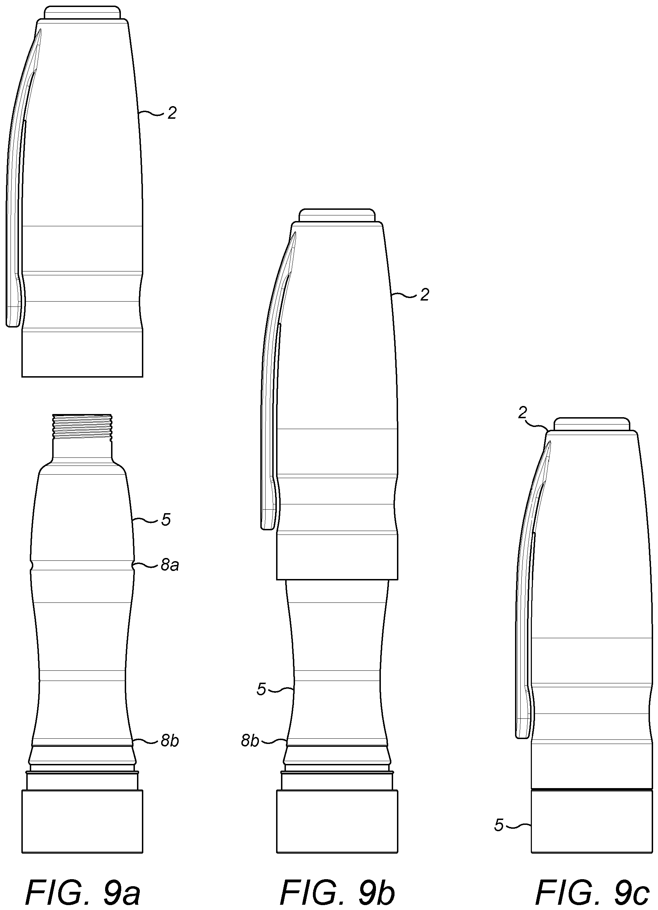

Figures 9a, 9b and 9c show a cap 2 in three different configurations. Figure 9a shows the cap 2 removed from the housing 5. Figure 9b shows the cap 2 in a raised position on the housing 5. Figure 9c shows the cap 2 in a lowered position on the housing 5. The cap 2 is the same or similar to that of figures 1 and 2; in particular in this case the cap is a snap fit onto the housing 5 although the protrusions 7 or other co-operating features are not shown in figure 9. The housing 5 has two snap fit grooves 8a and 8b. With the protrusions 7 located in groove 8a in figure 9b the cap is held in a raised position. When a needle is not located on the end 6 of the housing 5, then the cap can be pushed further onto the housing until the protrusions 7 are located in the lower groove 8b as showing figure 9c.

Alternatively the cap could have upper and lower feature 7, co-operating with a single co-operating feature 8a on the housing to achieve the same effect.

Furthermore, a screw attachment for a single cap could allow the cap to be fixed in two different positions dependent on whether a needle cartridge is present on the injector housing.

Embodiments of the invention have been described. Variations and modifications will suggest themselves to those skilled in the art without departing from the scope of the inventions as defined by the appended claims.

## Claims

1. A re-usable, multi-dose injection device having a housing (5) which is suitable for a receiving a medicament cartridge, and also suitable for receiving a needle (9), and comprising two interchangeable caps (2a, 2b) that can be remove-ably attached to a needle receiving end of the housing (5), **characterized in that** one cap (2a) is longer than the other (2b).

2. An injection device as claimed in claim 1, wherein the two caps (2a, 2b) use the same mechanical means to attach to the needle receiving end of the device.

3. An injection device as claimed in claim 1, wherein the two caps (2a, 2b) use different mechanical means to attach to the needle receiving end of the device.

4. An injection device as claimed in claim 2 or 3, wherein at least one cap (2a, 2b) uses a snap fit to attach to the needle receiving end of the device.

5. An injection device as claimed in claim 2, 3 or 4, wherein at least one cap (2a, 2b) uses a co-operating screw thread arrangement to attach to the needle receiving end of the device.

## Patentansprüche

1. Wiederverwendbare, Mehrfachinjektionsvorrichtung mit einem Gehäuse (5), das für die Aufnahme einer Medikamentenkartusche geeignet ist und ebenfalls für die Aufnahme einer Nadel (9) geeignet ist, und zwei austauschbare Kappen (2a, 2b) umfassend, die abnehmbar an einem Nadelaufnahmeende des Gehäuses (5) befestigt werden können, **dadurch gekennzeichnet, dass**
eine Kappe (2a) länger als die andere (2b) ist.

2. Injektionsvorrichtung nach Anspruch 1, worin die beiden Kappen (2a, 2b) die gleichen mechanischen Mittel verwenden, um an dem Nadelaufnahmeende der Vorrichtung befestigt zu werden.

3. Injektionsvorrichtung nach Anspruch 1, worin die beiden Kappen (2a, 2b) unterschiedliche mechanische Mittel verwenden, um an dem Nadelaufnahmeende der Vorrichtung befestigt zu werden.

4. Injektionsvorrichtung nach Anspruch 2 oder 3, wobei mindestens eine Kappe (2a, 2b) einen Schnappverbindung verwendet, um an dem Nadelaufnahmeende der Vorrichtung befestigt zu werden.

5. Injektionsvorrichtung nach Anspruch 2, 3 oder 4, wobei mindestens eine Kappe (2a, 2b) eine zusammenwirkende Schraubengewindeanordnung verwendet, um sie an dem Nadelaufnahmeende der Vorrichtung zu befestigen.

## Revendications

1. Dispositif d'injection multidose réutilisable comportant un boîtier (5) adapté pour recevoir une cartouche de médicament et également adapté pour recevoir une aiguille (9), et comprenant deux capuchons interchangeables (2a, 2b) pouvant être fixé de manière amovible à une extrémité du boîtier (5) recevant l'aiguille, **caractérisé en ce que**
l'un des capuchons (2a) est plus long que l'autre (2b).

2. Dispositif d'injection selon la revendication 1, dans lequel les deux capuchons (2a, 2b) utilisent les mêmes moyens mécaniques pour se fixer à l'extrémité du dispositif recevant l'aiguille.

3. Dispositif d'injection selon la revendication 1, dans lequel les deux capuchons (2a, 2b) utilisent des moyens mécaniques différents pour se fixer à l'extrémité du dispositif recevant l'aiguille.

4. Dispositif d'injection selon la revendication 2 ou 3, dans lequel au moins un capuchon (2a, 2b) utilise un ajustement à pression pour se fixer à l'extrémité du dispositif recevant l'aiguille.

5. Dispositif d'injection selon la revendication 2, 3 ou 4, dans lequel au moins un capuchon (2a, 2b) utilise un agencement de filetage de vis coopérant pour se fixer à l'extrémité du dispositif recevant l'aiguille.
